# EUROPÄISCHE PATENTSCHRIFT

(11) **EP 1 415 604 B1**
(45) Veröffentlichungstag und Bekanntmachung des Hinweises auf die Patenterteilung: **09.07.2008**
(21) Anmeldenummer: 02024606.2
(22) Anmeldetag: 04.11.2002
(51) Int. Cl.: A61B 17/72

(54) **Knochenfixierungssystem**
Bone fixationsystem
Système de fixation d'os

(43) Veröffentlichungstag der Anmeldung: 06.05.2004
(73) Patentinhaber: Zimmer GmbH, 8404 Winterthur (CH)
(72) Erfinder: Adam, Michael, 6402 Merlischchen (CH)
(74) Vertreter: Manitz, Finsterwald & Partner GbR

(56) Entgegenhaltungen:
- DE-U- 20 012 877
- US-A- 4 827 917

## Beschreibung

Die Erfindung betrifft ein Knochenfixierungssystem gemäß Oberbegriff des Anspruchs 1.

Derartige Systeme dienen insbesondere zur Reposition distaler Femurfrakturen, wie sie beispielsweise im "Manual der Osteosynthese", 3. Auflage, Springer-Verlag, Autoren: M. E. Müller, M. Allgöwer, R. Schneider, H. Willenegger, auf Seite 141 dargestellt sind. Bei dem Nagel handelt es sich insbesondere um einen Femurmarknagel, der vom Kniegelenk her eingesetzt wird und auf den eine Zielvorrichtung aufgesetzt werden kann, die es gestattet, mittels eines Bohrers die im Nagel ausgebildeten Querbohrungen in dem durch die Zielvorrichtung vorgegebenen, der Orientierung der Querbohrungen entsprechenden Winkel zu treffen und im Knochen Vorbohrungen für die anschließend einzusetzenden Schrauben auszubilden. Diese insbesondere in Form von Kondylenschrauben vorgesehenen Schrauben dienen dazu, den Marknagel bezüglich seiner axialen Richtung im Knochen zu fixieren.

Ein derartiges Fixierungssystem kommt auch dann zum Einsatz, wenn mittels der durch die Querbohrungen des Nagels gesteckten Schrauben Kondylenfragmente zum Restknochen hin fixiert werden sollen. Insbesondere dann, wenn sich die jeweilige Fraktur durch eine Mehrzahl von Kondylenfragmenten auszeichnet, stellt der Marknagel, der im Restknochen gehalten und mittels dortiger Verriegelungsschrauben fixiert ist, die einzige feste Bezugsbasis für die Fixierung der Kondylenfragmente dar. Beispiele für derartige Frakturen sind in dem vorstehend erwähnten "Manual der Osteosynthese" auf Seite 141 in den Abbildungen C1, C2 und C3 gezeigt.

Als problematisch kann sich bei derartigen Frakturen erweisen, dass die zur Fixierung der Kondylenfragmente dienenden, durch die Querbohrungen des Marknagels hindurchgeführten Knochenschrauben bezüglich ihrer eigenen Axialrichtung relativ zum Marknagel im Wesentlichen frei beweglich sind.

Aus der DE 200 12 877 U1 ist ein Verriegelungsnagel bekannt, der eine in Längsrichtung länglich geformte Querbohrung für eine Knochenschraube aufweist. Die Knochenschraube kann in der Querbohrung in axialer Richtung des Verriegelungsnagels verstellt werden, und zwar mittels eines Verriegelungselementes, das einen Außengewindeabschnitt aufweist und mit einem Innengewinde des im Bereich der länglichen Querbohrung hohl ausgeführten Verriegelungsnagels verschraubbar ist.

In der DE 296 20 327 U1 wird ebenfalls das Ziel verfolgt, die Position und die Orientierung von Verriegelungsschrauben bezüglich eines Verriegelungsnagels verändern zu können. Hierzu ist der Verriegelungsnagel mit einer sich in Längsrichtung des Nagelschaftes erstreckenden länglichen Öffnung versehen. Ferner sind so genannte Kulissensteine in den Nagelschaft einbringbar, wobei die Zwischenräume zwischen den Kulissensteinen als Durchgänge für die Verriegelungsschrauben dienen.

Im Gegensatz zu diesem Stand der Technik geht die Erfindung von einem Knochenfixierungssystem mit winkelstabilen Schrauben aus, deren Orientierung und Position jeweils durch die Querbohrung des Nagels festgelegt sind.

US 4,827,917 (auf dessen System der Oberbegriff des Anspruchs 1 basiert) und US 6,406,477 beschreiben Systeme zur Fixierung von Femurhalsfrakturen, wobei hierzu zwei parallel verlaufende Schrauben eingesetzt werden können.

Aufgabe der Erfindung ist es, ein Knochenfixierungssystem der eingangs genannten Art derart weiterzubilden, dass der Nagel auf möglichst einfache und sichere Weise mittels der Schraube zuverlässig und dauerhaft fixiert werden kann, wobei es insbesondere möglich sein soll, bei der Reposition distaler Femurfrakturen eine Mehrzahl von Kondylenfragmenten zuverlässig in ihrer korrekten Position zum Restknochen hin zu fixieren.

Die Lösung dieser Aufgabe erfolgt durch die Merkmale des Anspruchs 1.

Erfindungsgemäß kann einfach durch Verstellen eines in die Längsbohrung des Nagels eingebrachten Klemmorgans die durch die Querbohrung des Nagels gesteckte Schraube in der jeweils gewünschten Durchstecktiefe relativ zum Nagel festgesetzt und auf diese Weise bezüglich ihrer Axialrichtung fixiert werden, und zwar unter Beibehaltung ihrer durch die Querbohrung des Nagels festgelegten Orientierung und Position bezüglich der Längsachse des Nagels. Von besonderem Vorteil ist hierbei, dass die Durchstecktiefe der Schraube frei wählbar, d.h. die Schraube in jeder Durchstecktiefe mittels des Klemmorgans festsetzbar ist.

Vorteilhaft ist ferner, dass das erfindungsgemäße Klemmorgan zusammen mit herkömmlichen Schrauben verwendbar ist, so dass keine speziell ausgebildeten Schrauben erforderlich sind. Des Weiteren ist erfindungsgemäß von Vorteil, dass eine im Nagel vorhandene Längsbohrung, mittels welcher der Nagel auf einem so genannten Kirschnerdraht geführt werden kann, gleichzeitig für das erfindungsgemäße Klemmorgan nutzbar ist.

In einer bevorzugten Ausführungsform der Erfindung ist die Längsbohrung des Nagels mit einem Innengewindeabschnitt versehen, mit dem das Klemmorgan verschraubbar ist. Hierbei kann das Festsetzen der Schraube einfach durch eine Schraubbewegung des Klemmorgans bewirkt werden.

In einer bevorzugten praktischen Ausgestaltung ist das Klemmorgan einteilig ausgebildet und insbesondere in Form einer Madenschraube vorgesehen.

In einer weiteren bevorzugten Ausführungsform der Erfindung ist in die Längsbohrung des Nagels wenigstens ein hülsen- oder buchsenartiger Einsatz eingebracht, der wenigstens einen mit der Querbohrung des Nagels ausgerichteten Durchgang aufweist und mit dem das Klemmorgan zusammenwirkt. Vorzugsweise ist die Innenseite des Einsatzes mit einem Innengewindeabschnitt versehen, mit dem das Klemmorgan verschraubbar ist.

Hierdurch wird ein direktes Zusammenwirken zwischen dem Klemmorgan einerseits und dem Nagel andererseits vermieden. Dies ist insbesondere dann von Vorteil, wenn der Nagel aus einem Material hergestellt ist, für das die Herstellung und der Gebrauch eines Gewindes für das Klemmorgan insbesondere wegen einer zu geringen Materialhärte problematisch ist. Für das Material des Nagels kommt aufgrund der guten Körperverträglichkeit bevorzugt Titan oder eine Titanlegierung in Frage. Ausbildung und Gebrauch eines Gewindes sind bei derartigen, vergleichsweise weichen Materialien vor allem bei relativ langen und gleichzeitig feinen Gewinden problematisch, wie sie für erfindungsgemäß zum Einsatz kommen können.

Aus diesem Grund wird gemäß einer weiteren Ausführungsform der Einsatz aus einem Material hergestellt, das eine höhere Zähfestigkeit und/oder Härte aufweist als das Material des Nagels. Für das Material des Einsatzes kommt beispielsweise eine Kobalt-Chrom-Legierung in Frage.

Bevorzugt wird der Einsatz drehfest mit dem Nagel verbunden. Vorzugsweise wird der Einsatz in die Längsbohrung des Nagels eingepresst oder eingeschraubt.

Gemäß einem weiteren bevorzugten Ausführungsbeispiel der Erfindung sind in dem Nagel mehrere Querbohrungen ausgebildet, wobei für jede durch eine der Querbohrungen hindurchführbare Schraube ein Klemmorgan vorgesehen ist. Hierdurch kann für die einzelnen Schrauben eine individuell einstellbare Klemmung realisiert werden.

Des Weiteren ist bevorzugt vorgesehen, dass ein Satz von unterschiedliche axiale Abstände zwischen den Querbohrungen aufweisenden Nägeln vorgesehen ist und die axiale Länge der Klemmorgane kleiner ist als der kleinste in dem Satz vorkommende axiale Abstand zwischen zwei aufeinander folgenden Querbohrungen.

Es hat sich gezeigt, dass auch mit vergleichsweise kurzen Klemmorganen eine sichere und dauerhafte Fixierung der Schrauben in den Querbohrungen des Nagels erreicht werden kann. Hierdurch ist es möglich, für den gesamten Satz von Nägeln lediglich eine Längensorte von Klemmorganen vorzusehen und dabei auch Nägel mit vergleichsweise geringen axialen Abständen zwischen aufeinander folgenden Querbohrungen zu verwenden.

In einer weiteren Ausführungsform der Erfindung ist vorgesehen, dass durch Ziehen am Klemmorgan mittels einer Verstelleinrichtung ein auf der der Verstelleinrichtung abgewandten Seite der Schraube gelegener Abschnitt des Klemmorgans gegen die Schraube bewegbar ist.

In dieser Variante der Erfindung ist folglich eine Verstelleinrichtung für das Klemmorgan vorgesehen, mit welcher das Klemmorgan gegen die Schraube gezogen werden kann.

Hierbei ist vorzugsweise das Klemmorgan in der Längsbohrung des Nagels zumindest in axialer Richtung frei beweglich und mit wenigstens einem mit der Querbohrung des Nagels ausrichtbaren Durchgang für die Schraube versehen. Bevorzugt ist das Klemmorgan hülsenförmig ausgebildet.

Eine besonders einfache Betätigung des Klemmorgans wird erreicht, wenn gemäß einem weiteren Ausführungsbeispiel der Erfindung die Verstelleinrichtung eine Zugschraube umfasst, die mit einem Gewindeabschnitt des Klemmorgans zusammenwirkt und zum Ziehen des Klemmorgans in axialer Richtung am Nagel abgestützt ist. Hierbei kann durch Drehen der Zugschraube das Klemmorgan in Richtung der Zugschraube gezogen und dabei gegen die durch die Querbohrung des Nagels gesteckte Schraube bewegt werden, um die Schraube festzuklemmen.

Vorzugsweise ist vorgesehen, dass das Klemmorgan mehrere in axialer Richtung voneinander beabstandete, jeweils mit einer Querbohrung des Nagels ausrichtbare Durchgänge aufweist. Hierbei verlaufen die Schrauben jeweils gleichzeitig durch die im Nagel ausgebildete Querbohrung und durch den im Klemmorgan vorgesehenen Durchgang, so dass durch eine axiale Verstellbewegung des Klemmorgans, insbesondere durch Ziehen am Klemmorgan mittels einer Zugschraube, eine Mehrzahl von Schrauben durch ein einziges Klemmorgan festgesetzt werden können.

Das Festsetzen aller Schrauben wird auf besonders einfache und gleichzeitig zuverlässige Weise sichergestellt, wenn gemäß einer weiteren bevorzugten Ausführungsform der Erfindung das Klemmorgan mittels der Verstelleinrichtung in axialer Richtung verformbar ist. Hierdurch ist es nicht erforderlich, dass alle Schrauben exakt zum gleichen Zeitpunkt mit dem Klemmorgan in Eingriff gelangen, um sicher zwischen dem Klemmorgan und der die jeweilige Bohrung begrenzenden Innenwand des Nagels festgeklemmt zu werden. Diese durch die axiale Verformbarkeit des Klemmorgans erzielte Toleranz gewährleistet, dass alle Schrauben mit einer ausreichend großen Klemmkraft in ihrer Querbohrung festgesetzt werden.

Ferner ist erfindungsgemäß vorzugsweise wenigstens ein Sicherungselement vorgesehen, das von außen durch die Seitenwand des Nagels hindurch in dessen Längsbohrung hinein bewegbar ist und mit dem das Klemmorgan in seiner Ausgangslage relativ zum Nagel vor Betätigung der Verstelleinrichtung fixierbar ist.

Das insbesondere in Form einer in die Seitenwand des Nagels einschraubbaren Sicherungsschraube vorgesehene Sicherungselement kann dabei derart ausgeführt sein, dass es durch die anschließende axiale Verstellbewegung des Klemmorgans verformt oder abgeschert wird, so dass das Sicherungselement einerseits eine korrekte Ausrichtung der Querbohrungen des Nagels mit den Durchgängen des Klemmorgans zum Hindurchführen der Schrauben gewährleistet, andererseits aber die anschließende axiale Verstellbewegung des Klemmorgans zum Festsetzen der Schrauben nicht stört.

Weitere Ausführungsformen der Erfindung sind in den abhängigen Ansprüchen, der Beschreibung sowie der Zeichnung angegeben.

Die Erfindung wird im Folgenden beispielhaft unter Bezugnahme auf die Zeichnung beschrieben. Es zeigen.
- Fig. 1: ein Knochenfixierungssystem gemäß einer ersten Ausführungsform der Erfindung,
- Fig. 2: verschiedene Ansichten eines mit mehreren Klemmorganen bestückten Nagels des Systems von Fig. 1,
- Fig. 3: ein Klemmorgan des Systems von Fig. 1,
- Fig. 4: verschiedene Ansichten eines Knochenfixierungssystems gemäß einer zweiten Ausführungsform der Erfindung,
- Fig. 5: verschiedene Ansichten eines Nagels des Systems von Fig. 4,
- Fig. 6: verschiedene Ansichten eines hülsenförmigen Einsatzes des Systems von Fig. 4,
- Fig. 7: ein Klemmorgan des Systems von Fig. 4,
- Fig. 8: verschiedene Ansichten eines Knochenfixierungssystems gemäß einer dritten Ausführungsform der Erfindung,
- Fig. 9: verschiedene Ansichten eines Nagels des Systems von Fig. 8,
- Fig. 10: verschiedene Ansichten eines hülsenförmigen Klemmorgans des Systems von Fig. 8,
- Fig. 11: ein Sicherungselement des Systems von Fig. 8, und
- Fig. 12: verschiedene Ansichten des Systems von Fig. 8 ohne Kondylenschrauben im zusammengesetzten Zustand.

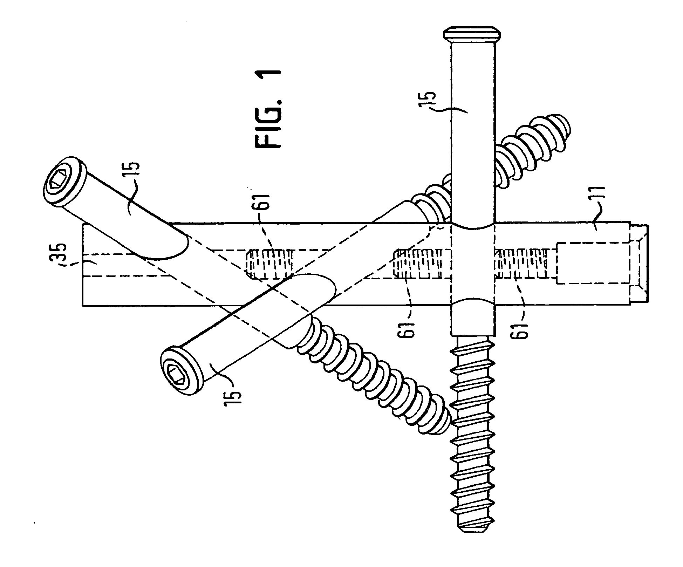

Das in den Fig. 1 bis 3 dargestellte erfindungsgemäße Knochenfixierungssystem umfasst einen Femurmarknagel 11 und mehrere Kondylenschrauben 15, die durch im Nagel 11 ausgebildete Querbohrungen hindurchgeführt sind. Der Nagel 11 weist eine zentrale Längsbohrung 35 auf, die mit einem Innengewinde 36 versehen ist, über welches einzelne Klemmorgane 61 in Form von Madenschrauben in die Längsbohrung 35 des Nagels 11 hineingeschraubt sind, um die Kondylenschrauben 15 jeweils in ihrer gewünschten axialen Position zu fixieren.

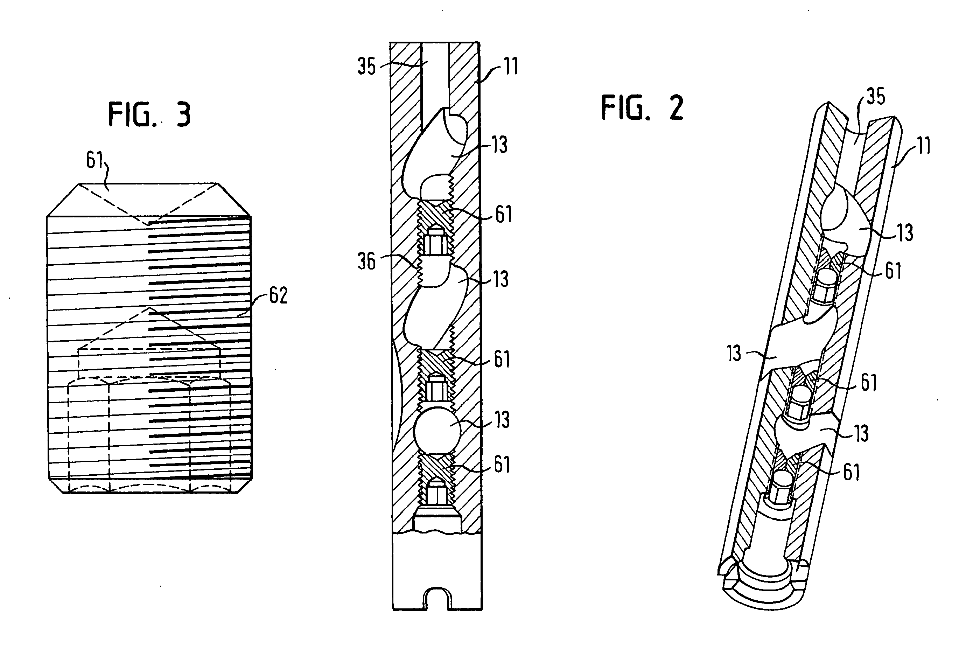

In der den zusammengesetzten Zustand im Schnitt ohne die Kondylenschrauben zeigenden Fig. 2 ist insbesondere das Innengewinde 36 des Nagels 11 gezeigt, mit dem die die Längsbohrung 35 des Nagels 11 begrenzende Innenwand versehen ist.

Die in Fig. 3 relativ zu Fig. 2 vergrößert dargestellte, mit einem entsprechenden Außengewinde 62 versehene Madenschraube 61 ist mittels eines Sechskantwerkzeugs betätigbar.

Die Madenschrauben 61 werden über das in Fig. 2 untere Ende des Nagels 11 in dessen Längsbohrung 35 eingeführt und mittels des erwähnten Werkzeugs eingeschraubt. In dieser Variante der Erfindung ist die Reihenfolge des Festsetzens der Kondylenschrauben 15 vorgegeben, da zuerst die jeweils am weitesten von dem Einführende entfernt gelegene Kondylenschraube mittels des betreffenden Klemmorgans 61 fixiert werden muss, bevor eine weitere Kondylenschraube 15 in die nächstgelegene Querbohrung 13 des Nagels 11 eingeführt werden kann.

Wenn zum Einsetzen des Nagels 11 in den Knochen der Nagel 11 nicht auf einem so genannten Kirschnerdraht geführt wird, dann kann die innerste, d.h. die in Fig. 2 oberste Madenschraube 61 bereits in den Nagel 11 eingeschraubt sein, wenn dieser in den Knochen eingesetzt wird.

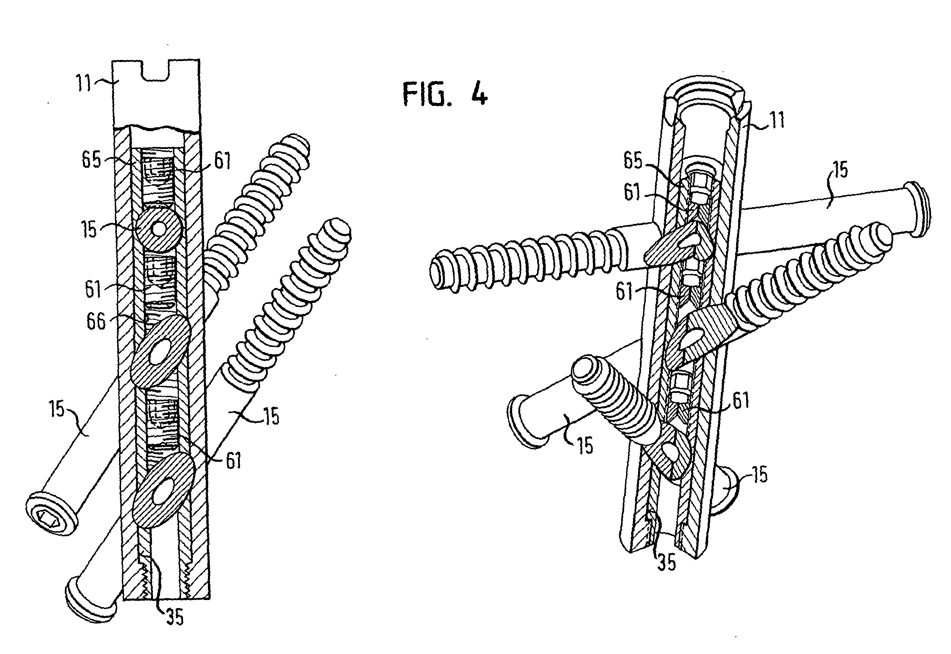

Die in den Fig. 4 bis 7 dargestellte zweite Ausführungsform der Erfindung unterscheidet sich von der vorstehend erläuterten Variante dadurch, dass in die Längsbohrung 35 des Nagels 11 ein hülsenförmiger Einsatz 65 eingebracht ist. Der Hülseneinsatz 65 kann beispielsweise in den Nagel 11 eingepresst oder eingeschraubt sein.

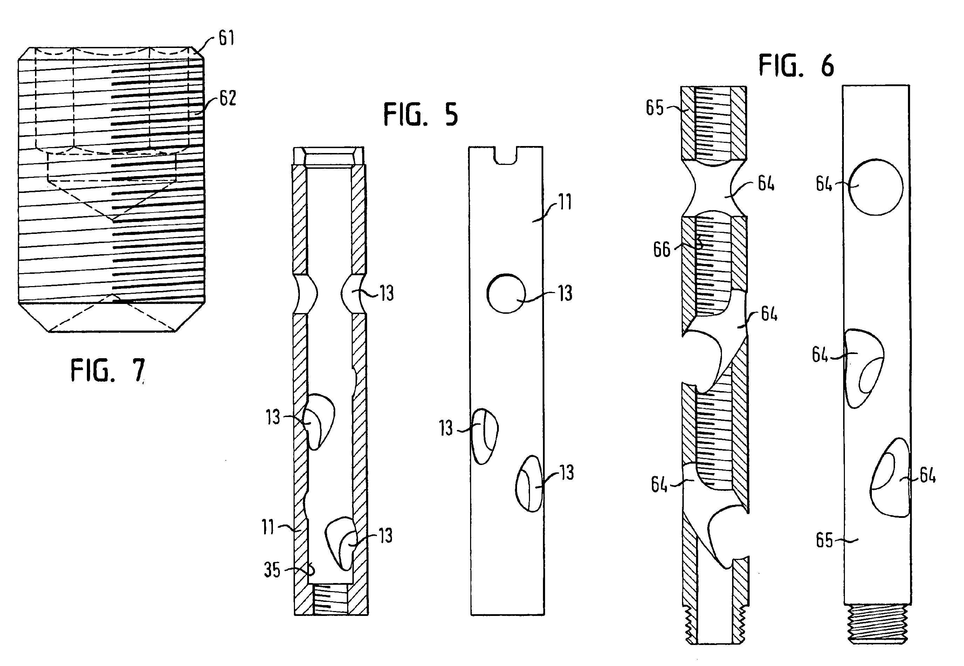

Wie Fig. 6, in welcher der Hülseneinsatz 65 gegenüber dem in Fig. 5 dargestellten Nagel 11 vergrößert dargestellt ist, zeigt, ist der Einsatz 65 mit Durchgängen 64 für die Kondylenschrauben 15 versehen, wobei im in die Längsbohrung 35 des Nagels 11 eingebrachten Zustand gemäß Fig. 4 die Durchgänge 64 des Einsatzes 65 mit den Querbohrungen 13 des Nagels ausgerichtet sind.

Die Innenwand des Hülseneinsatzes 65 ist mit einem Innengewindeabschnitt 66 versehen, in den die Madenschrauben 61 (vgl. Fig. 7) eingeschraubt werden, um die sich jeweils durch einen Durchgang des Einsatzes 65 und eine Querbohrung 13 des Nagels 11 hindurch erstreckenden Kondylenschrauben 15 zu fixieren.

Was die Reihenfolge des Festsetzens der Schrauben 15 anbetrifft, so gilt diesbezüglich das in Verbindung mit der ersten Ausführungsform gemäß Fig. 1 bis 3 Gesagte.

Der Hülseneinsatz 65 besteht vorzugsweise aus einem Material, das zähfester bzw. härter Material ist als das Material des Nagels 11. Während der Nagel 11 vorzugsweise aus Titan oder einer Titanlegierung und damit aus einem vergleichsweise weichen Material hergestellt ist, besteht der Hülseneinsatz 65 bevorzugt aus einer Kobalt-Chrom-Legierung.

Prinzipiell kommen erfindungsgemäß auch andere Materialkombinationen in Frage.

Durch die Verwendung des Hülseneinsatzes 65 wird ein direktes Zusammenwirken zwischen den Madenschrauben 61 und dem Nagel 11 vermieden, wodurch unabhängig von dem für den Nagel 11 verwendeten Material eine stabile und zuverlässige Fixierung der Kondylenschrauben 15 durch die Madenschrauben 61 dauerhaft gewährleistet ist.

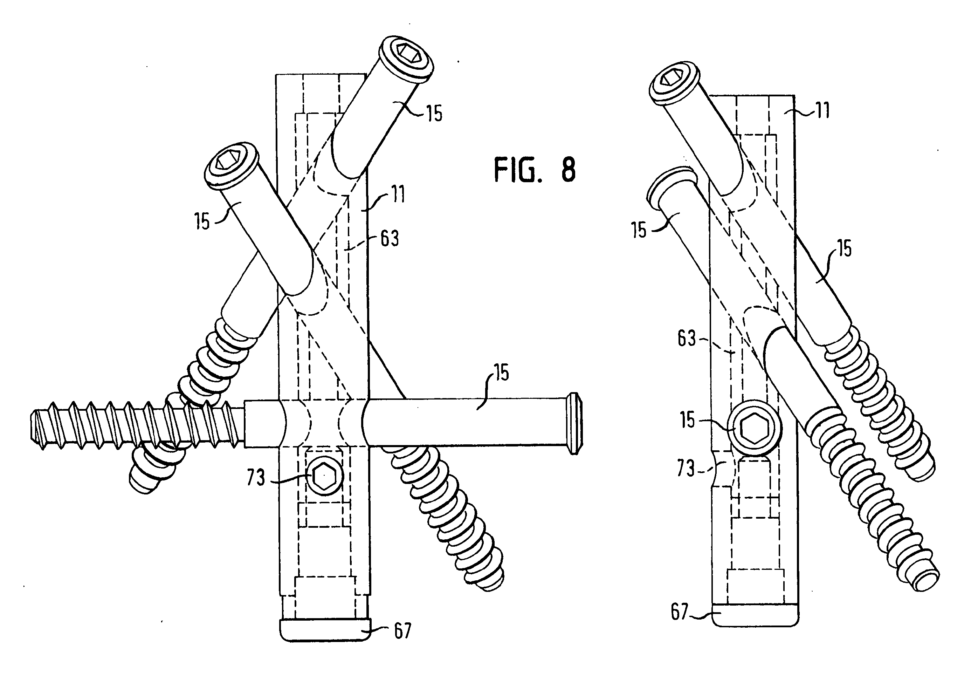

Anders als bei den zuvor erläuterten Varianten ist bei der in den Fig. 8 bis 12 dargestellten dritten Ausführungsform der Erfindung für mehrere Kondylenschrauben 15 ein einziges gemeinsames Klemmorgan 63 in Form einer Klemmhülse 63 vorgesehen, mit der die Kondylenschrauben 15 durch eine einzige axiale Verstellbewegung der Klemmhülse 63 und damit praktisch gleichzeitig fixiert werden können.

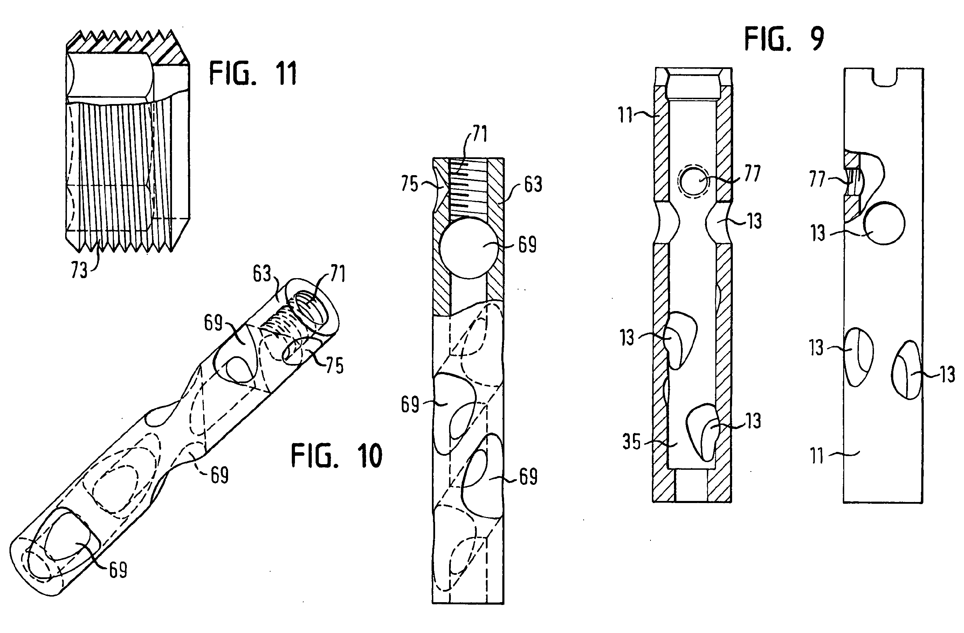

Die in Fig. 10 gegenüber dem in Fig. 9 dargestellten Nagel 11 vergrößert gezeigte Klemmhülse 63 ist in die Längsbohrung 35 des Nagels 11 einführbar und darin axial frei beweglich. Die Klemmhülse 63 ist mit Durchgängen 69 versehen, die mit den im Nagel 11 ausgebildeten Querbohrungen 13 ausgerichtet werden können. Die korrekte Ausrichtung sowohl bezüglich der axialen Position als auch bezüglich der Drehstellung der Klemmhülse 63 relativ zum Nagel 11 wird durch ein in Fig. 11 vergrößert dargestelltes Sicherungselement 73 in Form einer Sicherungsschraube gewährleistet.

Die Seitenwand des Nagels 11 ist mit einem Durchgang 77 für die Sicherungsschraube 73 versehen, der einen Innengewindeabschnitt aufweist, mit dem das ein entsprechendes Außengewinde aufweisende Sicherungselement 73 derart verschraubt werden kann, dass das Sicherungselement 73 in die Längsbohrung 35 des Nagels 11 hinein vorsteht und mit einer für das Sicherungselement 73 vorgesehenen Vertiefung 75 der Klemmhülse 63 in Eingriff gelangt.

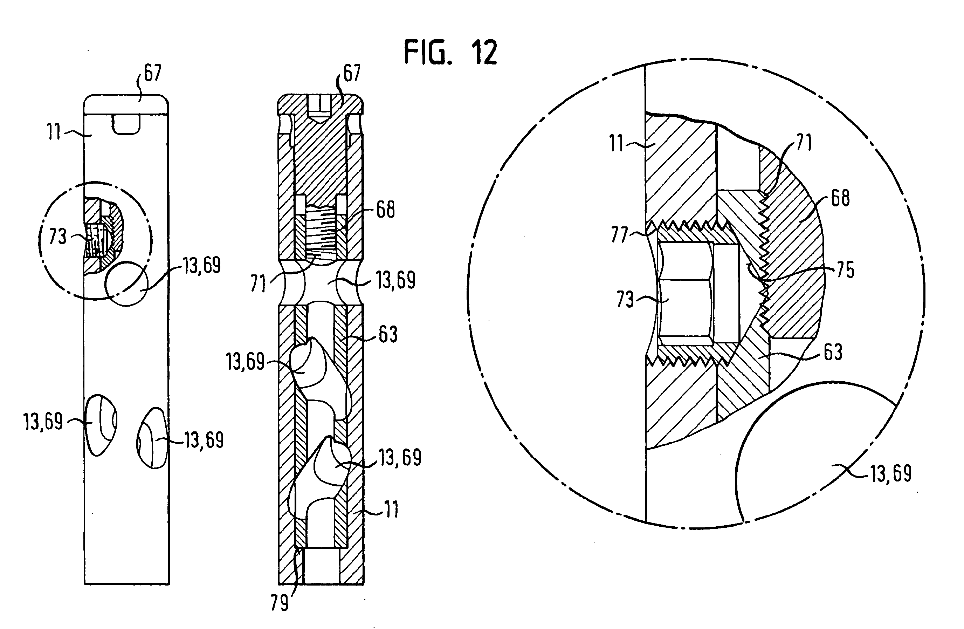

Die zur korrekten Ausrichtung der Klemmhülse 63 relativ zum Nagel 11 dienende Sicherung ist insbesondere in der vergrößerten Darstellung ganz rechts in Fig. 12 gezeigt.

Die axiale Verstellbewegung der Klemmhülse 63 zum gleichzeitigen Festsetzen der durch die Querbohrungen 13 des Nagels 11 und die Durchgänge 69 der Klemmhülse 63 hindurchgeführten Kondylenschrauben 15 (vgl. Fig. 8) wird durch eine Verstelleinrichtung 67 in Form einer Zugschraube bewirkt, die über einen mit einem Außengewinde versehenen Zugabschnitt 68 mit einem Innengewindeabschnitt 71 der Klemmhülse 63 (vgl. Fig. 10) verschraubt ist und sich mit ihrem Kopf an der Stirnseite des Nagels 11 abstützt.

Durch Drehen der Zugschraube 67 wird die Klemmhülse 63 in Richtung der Zugschraube 67 gezogen. Hierdurch werden alle Kondylenschrauben 15 im Wesentlichen gleichzeitig zwischen einem jeweiligen Klemmabschnitt der Klemmhülse 63 einerseits und der die jeweilige Querbohrung 13 begrenzenden Innenwand des Nagels 11 andererseits eingeklemmt und damit fixiert.

Die vorzugsweise aus Kunststoff hergestellte Sicherungsschraube 73 ist derart ausgebildet, dass sie durch diese axiale Verstellbewegung der Klemmhülse 63 verformt oder abgeschert und somit das Festsetzen der Kondylenschrauben 15 durch die Sicherungsschraube 73 nicht beeinträchtigt wird. Die Sicherungsschraube 73 kann beispielsweise aus einem körperverträglichen, resorbierbaren Polylactat bestehen.

Um zu gewährleisten, dass alle Kondylenschrauben 15 sicher festgesetzt werden, ist die Klemmhülse 63 vorzugsweise aus einem Material hergestellt, das durch die mittels der Zugschraube 67 aufbringbaren Zugkräfte in axialer Richtung verformt werden kann.

Damit nach Beginn der Ziehbewegung der Klemmhülse 63 zuerst die am weitesten von der Zugschraube 67 entfernt gelegene Kondylenschraube 15 in Klemmeingriff mit der Klemmhülse 63 gelangt, können die Durchgänge 69 der Klemmhülse 63 derart positioniert werden, dass sie mit den Querbohrungen 13 des Nagels 11 in einem vorgespannten Zustand der Klemmhülse 63 ausgerichtet sind, in welchem die Klemmhülse 63 gegen den axialen Endanschlag 79 des Nagels 11 gedrückt und in axialer Richtung zumindest geringfügig zusammengestaucht ist.

Ein Vorteil der für mehrere Schrauben 15 vorgesehenen Klemmhülse 63 besteht darin, dass die Klemmung der Kondylenschrauben 15 auf besonders einfache Weise wieder gelöst werden kann, indem die Zugschraube 67 ein Stück weit herausgedreht und dadurch die Klemmhülse 63 etwas entlastet wird, woraufhin durch einen leichten Schlag auf den Kopf der Zugschraube 67 alle Klemmverbindungen zwischen der Klemmhülse 63 und den Kondylenschrauben 15 gleichzeitig gelöst werden.

### Bezugszeichenliste

- 11: Nagel
- 13: Querbohrung
- 15: Schraube
- 35: Längsbohrung des Nagels
- 36: Innengewindeabschnitt des Nagels
- 61: Klemmorgan, Madenschraube
- 62: Außengewinde der Madenschraube
- 63: Klemmorgan, Klemmhülse
- 64: Durchgang des Einsatzes
- 65: Einsatz
- 66: Innengewindeabschnitt des Einsatzes
- 67: Verstelleinrichtung, Zugschraube
- 68: Zugabschnitt der Verstelleinrichtung
- 69: Durchgang der Klemmhülse
- 71: Gewindeabschnitt der Klemmhülse
- 73: Sicherungselement, Sicherungsschraube
- 75: Vertiefung der Klemmhülse
- 77: Durchgang des Nagels
- 79: Endanschlag des Nagels

## Patentansprüche

1. Knochenfixierungssystem mit wenigstens einem Nagel (11), insbesondere einem Femurmarknagel, und zumindest einer Schraube (15), insbesondere einer Kondylenschraube, die durch eine im Nagel (11) ausgebildete, die Orientierung und die Position der Schraube (15) bezüglich der Längsachse des Nagels (11) festlegende Querbohrung (13) hindurchführbar ist, wobei
wenigstens ein Klemmorgan (61, 63) vorgesehen ist, das in eine Längsbohrung (35) des Nagels (11) einbringbar und in der Längsbohrung (35) relativ zum Nagel (11) axial verstellbar ist, wobei durch die Verstellbewegung des Klemmorgans (61, 63) die durch die Querbohrung (13) des Nagels (11) hindurchgeführte Schraube (15) zwischen dem Klemmorgan (61, 63) und der die Querbohrung (13) begrenzenden Innenwand des Nagels (11) festklemmbar ist,
**dadurch gekennzeichnet,**
**dass** im Nagel (11) drei in Richtung der Längsachse des Nagels (11) beabstandete Querbohrungen (13) für jeweils eine Schraube (15) ausgebildet sind, wobei sich die von den Querbohrungen (13) festgelegten Orientierungen und Positionen der Schrauben (15) jeweils paarweise voneinander unterscheiden und die durch die drei Querbohrungen (13) hindurchgeführten Schrauben (15) durch die Verstellbewegung des wenigstens einen Klemmorgans (61,63) festklemmbar sind.

2. Knochenfixierungssystem nach Anspruch 1,
**dadurch gekennzeichnet,**
**dass** die Längsbohrung (35) des Nagels (11) mit einem Innengewindeabschnitt (36) versehen ist, mit dem das Klemmorgan (61) verschraubbar ist.

3. Knochenfixierungssystem nach Anspruch 1 oder 2,
**dadurch gekennzeichnet,**
**dass** das Klemmorgan (61) einteilig ausgebildet und insbesondere in Form einer Madenschraube vorgesehen ist.

4. Knochenfixierungssystem nach einem der vorhergehenden Ansprüche,
**dadurch gekennzeichnet,**
**dass** in die Längsbohrung (35) des Nagels (11) wenigstens ein hülsen- oder buchsenartiger Einsatz (65) eingebracht ist, der wenigstens einen mit der Querbohrung (13) des Nagels (11) ausgerichteten Durchgang (64) aufweist und mit dem das Klemmorgan (61) zusammenwirkt.

5. Knochenfixierungssystem nach Anspruch 4,
**dadurch gekennzeichnet,**
**dass** die Innenseite des Einsatzes (65) mit einem Innengewindeabschnitt (66) versehen ist, mit dem das Klemmorgan (61) verschraubbar ist.

6. Knochenfixierungssystem nach Anspruch 4 oder 5,
**dadurch gekennzeichnet,**
**dass** der Einsatz (65) aus einem Material, insbesondere einer Kobalt-Chrom-Legierung, hergestellt ist, das eine höhere Zähfestigkeit und/oder Härte aufweist als das Material, insbesondere Titan oder eine Titanlegierung, des Nagels (11).

7. Knochenfixierungssystem nach einem der Ansprüche 4 bis 6,
**dadurch gekennzeichnet,**
**dass** der Einsatz (65) drehfest mit dem Nagel (11) verbunden ist.

8. Knochenfixierungssystem nach einem der Ansprüche 4 bis 7,
**dadurch gekennzeichnet,**
**dass** der Einsatz (65) in die Längsbohrung (35) des Nagels (11) eingepresst oder eingeschraubt ist.

9. Knochenfixierungssystem nach einem der vorhergehenden Ansprüche,
**dadurch gekennzeichnet,**
**dass** in dem Nagel (11) drei oder mehr Querbohrungen (13) ausgebildet sind und für jede durch eine der Querbohrungen (13) hindurchführbare Schraube (15) ein Klemmorgan (61) vorgesehen ist.

10. Knochenfixierungssystem nach Anspruch 9,
**dadurch gekennzeichnet,**
**dass** ein Satz von unterschiedliche axiale Abstände zwischen den Querbohrungen (13) aufweisenden Nägeln (11) vorgesehen ist und die axiale Länge der Klemmorgane (61) jeweils kleiner ist als der kleinste in dem Satz vorkommende axiale Abstand zwischen zwei aufeinander folgenden Querbohrungen (13).

11. Knochenfixierungssystem nach Anspruch 1,
**dadurch gekennzeichnet,**
**dass** durch Ziehen am Klemmorgan (63) mittels einer Verstelleinrichtung (67) ein auf der der Verstelleinrichtung (67) abgewandten Seite der Schraube (15) gelegener Abschnitt des Klemmorgans (63) gegen die Schraube (15) bewegbar ist.

12. Knochenfixierungssystem nach Anspruch 11,
**dadurch gekennzeichnet,**
**dass** das Klemmorgan (63) in der Längsbohrung (35) des Nagels (11) zumindest in axialer Richtung frei beweglich ist und wenigstens einen mit der Querbohrung (13) des Nagels (11) ausrichtbaren Durchgang (69) für die Schraube (15) aufweist, wobei vorzugsweise das Klemmorgan (63) hülsenförmig ausgebildet ist.

13. Knochenfixierungssystem nach Anspruch 11 oder 12,
**dadurch gekennzeichnet,**
**dass** die Verstelleinrichtung (67) eine Zugschraube umfasst, die mit einem Gewindeabschnitt (71) des Klemmorgans (63) zusammenwirkt und zum Ziehen des Klemmorgans (63) in axialer Richtung am Nagel (11) abgestützt ist.

14. Knochenfixierungssystem nach einem der Ansprüche 11 bis 13,
**dadurch gekennzeichnet,**
**dass** das Klemmorgan (63) mehrere in axialer Richtung voneinander beabstandete, jeweils mit einer Querbohrung (13) des Nagels (11) ausrichtbare Durchgänge (69) aufweist.

15. Knochenfixierungssystem nach einem der Ansprüche 11 bis 14,
**dadurch gekennzeichnet,**
**dass** das Klemmorgan (63) mittels der Verstelleinrichtung (67) in axialer Richtung verformbar ist.

16. Knochenfixierungssystem nach Anspruch 15,
**dadurch gekennzeichnet,**
**dass** das Klemmorgan (63) derart verformbar ist, dass durch die Verstellbewegung mehrere in axialer Richtung des Nagels (11) voneinander beabstandete Schrauben (15) jeweils zwischen dem Klemmorgan (63) und der die jeweilige Querbohrung (13) begrenzenden Innenwand des Nagels (11) festklemmbar sind.

17. Knochenfixierungssystem nach einem der Ansprüche 11 bis 16,
**dadurch gekennzeichnet,**
**dass** wenigstens ein von außen durch die Seitenwand des Nagels (11) hindurch in dessen Längsbohrung (35) hinein bewegbares Sicherungselement (73), insbesondere eine Sicherungsschraube, vorgesehen ist, mit dem das Klemmorgan (63) in seiner Ausgangslage relativ zum Nagel (11) vor Betätigung der Verstelleinrichtung (67) fixierbar ist.

## Claims

1. A bone fixing system comprising at least one nail (11), in particular a femoral medullary nail, and at least one screw (15), in particular a condyle screw, which can be guided through a transverse bore (13) formed in the nail (11) and defining the orientation and the position of the screw (15) with respect to the longitudinal axis of the nail (11), wherein
at least one clamping member (61, 63) is provided which can be introduced into a longitudinal bore (35) of the nail (11) and is axially adjustable in the longitudinal bore (35) relative to the nail (11), with the screw (15) guided through the transverse bore (13) of the nail (11) being able to be clamped between the clamping member (61, 63) and the inner wall of the nail (11) bounding the transverse bore (13) by the displacement movement of the clamping member (61, 63),
**characterized in that**
three transverse bores (3) for one respective screw (15) each are formed in the nail (11) and are spaced apart in the direction of the nail (11), with the orientations and positions of the screws (15) defined by the transverse bores (13) each differing from one another pair-wise and the screws (15) guided through the three transverse bores (13) being able to be clamped by the displacement movement of the at least one clamping member (61, 63).

2. A bone fixing system in accordance with claim 1, **characterized in that** the longitudinal bore (35) of the nail (11) is provided with an internal thread section (36) with which the clamping member (61) can be screwed.

3. A bone fixing system in accordance with claim 1 or claim 2, **characterized in that** the clamping member (61) is made in one piece and is in particular provided in the form of a grub screw.

4. A bone fixing system in accordance with any one of the preceding claims, **characterized in that** at least one sleeve-like or bushing-like insert (65) is introduced into the longitudinal bore (35) of the nail (11) and has at least one passage (64) aligned with the transverse bore (13) of the nail (11) and with which the clamping member (61) cooperates.

5. A bone fixing system in accordance with claim 4, **characterized in that** the inner side of the insert (65) is provided with an internal thread section (66) with which the clamping member (61) can be screwed.

6. A bone fixing system in accordance with claim 4 or claim 5, **characterized in that** the insert (65) is made of a material, in particular a cobalt chromium alloy, which has a higher toughness and/or hardness than the material, in particular titanium or a titanium alloy, of the nail (11).

7. A bone fixing system in accordance with any one of the claims 4 to 6, **characterized in that** the insert (65) is rotationally fixedly connected to the nail (11).

8. A bone fixing system in accordance with any one of the claims 4 to 7, **characterized in that** the insert (65) is pressed or screwed into the longitudinal bore (35) of the nail (11).

9. A bone fixing system in accordance with any one of the preceding claims, **characterized in that** three or more transverse bores (13) are formed in the nail (11) and a clamping member (61) is provided for each screw (15) which can be guided through one of the transverse bores (13).

10. A bone fixing system in accordance with claim 9, **characterized in that** a set of different axial spacings is provided between the nails (11) having transverse bores (13) and the axial length of the clamping members (61) is respectively smaller than the smallest axial spacing between two sequential transverse bores (13) occurring in the set.

11. A bone fixing system in accordance with claim 1, **characterized in that** a section of the clamping member (63) disposed on the side of the screw (15) remote from the displacement device (67) can be moved against the screw (15) by pulling at the clamping member (63) by means of a displacement device (67).

12. A bone fixing system in accordance with claim 11, **characterized in that** the clamping member (63) is freely movable at least in the axial direction in the longitudinal bore (35) of the nail (11) and has at least one passage (69) for the screw (15) which can be aligned with the transverse bore (13) of the nail (11), with the clamping member (63) preferably being made in sleeve shape.

13. A bone fixing system in accordance with claim 11 or claim 12, **characterized in that** the displacement device (67) includes a drawing screw which cooperates with a thread section (71) of the clamping member (63) and is supported at the nail (11) for the drawing of the clamping member (63) in the axial direction.

14. A bone fixing system in accordance with any one of the claims 11 to 13, **characterized in that** the clamping member (63) has a plurality of passages (69) which are spaced apart from one another in the axial direction and can each be aligned with a transverse bore (13) of the nail (11).

15. A bone fixing system in accordance with any one of the claims 11 to 14, **characterized in that** the clamping member (63) can be deformed in the axial direction by means of the displacement device (67).

16. A bone fixing system in accordance with claim 15, **characterized in that** the clamping member (63) can be deformed such that a plurality of screws (15) spaced apart from one another in the axial direction of the nail (11) can each be clamped between the clamping member (63) and the inner wall of the nail (11) bounding the respective transverse bore (13) by the displacement movement.

17. A bone fixing system in accordance with any one of the claims 11 to 16, **characterized in that** at least one securing member (73), in particular a securing screw, is provided which can be moved from the outside through the side wall of the nail (11) into its longitudinal bore (35) and by which the clamping member (63) can be fixed in its starting position relative to the nail (11) prior to the actuation of the displacement device (67).

## Revendications

1. Système de fixation pour os, comprenant au moins une broche (11), en particulier une broche médullaire pour fémur, et au moins une vis (15), en particulier une vis de condyle, qui peut être passée à travers un perçage transversal (13) ménagé dans la broche (11) et déterminant l'orientation et la position de la vis (15) par rapport à l'axe longitudinal de la broche (11), dans lequel
il est prévu au moins un organe de serrage (61, 63), susceptible d'être introduit dans un perçage longitudinal (35) de la broche (11) et réglable axialement dans le perçage longitudinal (35) par rapport à la broche (11), de sorte que par le mouvement de réglage de l'organe de serrage (61, 63), la vis (15) passée à travers le perçage transversal (13) de la broche (11) peut être coincée entre l'organe de serrage (61, 63) et la paroi intérieure de la broche (11) qui délimite le perçage transversal (13),
**caractérisé en ce que**
trois perçages transversaux (13), chacun pour une vis respective (15), sont ménagés dans la broche (11) et à distance en direction de l'axe longitudinal de la broche (11), de sorte que les orientations et les positions des vis (15) déterminées par les perçages transversaux (13) diffèrent mutuellement à chaque paire, et
les vis (15) passées à travers les trois perçages transversaux (13) peuvent être coincées par le mouvement de réglage dudit au moins un organe de serrage (61, 63).

2. Système de fixation pour os selon la revendication 1,
**caractérisé en ce que** le perçage longitudinal (35) de la broche (11) est pourvu d'un tronçon taraudé (36) dans lequel l'organe de serrage (61) peut être vissé.

3. Système de fixation pour os selon la revendication 1 ou 2,
**caractérisé en ce que** l'organe de serrage (61) est réalisé d'une seule pièce est prévu en particulier sous la forme d'une vis sans tête.

4. Système de fixation pour os selon l'une des revendications précédentes,
**caractérisé en ce qu'**au moins un insert en forme de douille ou de manchon (65) est introduit dans le perçage longitudinal (35) de la broche (11), ledit insert présentant au moins une traversée (64) alignée avec le perçage transversal (13) de la broche (11) et coopérant avec l'organe de serrage (61).

5. Système de fixation pour os selon la revendication 4,
**caractérisé en ce que** le côté intérieur de l'insert (65) est pourvu d'un tronçon taraudé (66) dans lequel l'organe de serrage (61) peut être vissé.

6. Système de fixation pour os selon la revendication 4 ou 5,
**caractérisé en ce que** l'insert (65) est fabriqué en un matériau, en particulier un alliage cobalt-chrome, qui présente une résistance et/ou une dureté plus élevée que le matériau, en particulier du titane ou un alliage de titane, de la broche (11).

7. Système de fixation pour os selon l'une des revendications 4 à 6,
**caractérisé en ce que** l'insert (65) est relié solidaire en rotation avec la broche (11).

8. Système de fixation pour os selon l'une des revendications 4 à 7,
**caractérisé en ce que** l'insert (65) est enfoncé à la presse ou vissé dans le perçage longitudinal (35) de la broche (11).

9. Système de fixation pour os selon l'une des revendications précédentes,
**caractérisé en ce que** trois ou plusieurs perçages transversaux (13) sont ménagés dans la broche (11), et **en ce qu'**il est prévu un organe de serrage (61) pour chaque vis (15) qui peut être passée à travers l'un des perçages transversaux (13).

10. Système de fixation pour os selon la revendication 9,
**caractérisé en ce qu'**il est prévu un jeu de broches (11) qui présentent des distances axiales différentes entre les perçages transversaux (13), et **en ce que** la longueur axiale des organes de serrage (66) est à chaque fois plus petite que la plus petite distance axiale présente dans le jeu entre deux perçages transversaux successifs (13).

11. Système de fixation pour os selon la revendication 1,
**caractérisé en ce qu'**en serrant l'organe de serrage (63) au moyen d'un dispositif de réglage (67), un tronçon de l'organe de serrage (63), situé du côté de la vis (15) détourné du dispositif de réglage (67), est déplaçable contre la vis (15).

12. Système de fixation pour os selon la revendication 11,
**caractérisé en ce que** l'organe de serrage (63) est librement mobile, au moins en direction axiale, dans le perçage longitudinal (35) de la broche (11) et présente au moins une traversée (69) pour la vis (15), qui peut être alignée avec le perçage transversal (13) de la broche (11), ledit organe de serrage (63) étant de préférence réalisé en forme de douille.

13. Système de fixation pour os selon la revendication 11 ou 12,
**caractérisé en ce que** le dispositif de réglage (63) comprend une vis de traction qui coopère avec un tronçon taraudé (71) de l'organe de serrage (63) et qui est soutenu en direction axiale sur la broche (11) pour le serrage de l'organe de serrage (63).

14. Système de fixation pour os selon l'une des revendications 11 à 13,
**caractérisé en ce que** l'organe de serrage (63) comprend plusieurs traversées (69) à distance les unes des autres en direction axiale, susceptibles d'être alignées à chaque fois avec un perçage transversal (13) de la broche (11).

15. Système de fixation pour os selon l'une des revendications 11 à 14,
**caractérisé en ce que** l'organe de serrage (63) est déformable en direction axiale au moyen du dispositif de réglage (67).

16. Système de fixation pour os selon la revendication 15,
**caractérisé en ce que** l'organe de serrage (63) est déformable de telle façon que, par le déplacement de réglage, plusieurs vis (15) à distance les unes des autres en direction axiale de la broche (11) peuvent être coincées chacune entre l'organe de serrage (63) et la paroi intérieure de la broche (11) qui délimite le perçage transversal respectif (13).

17. Système de fixation pour os selon l'une des revendications 11 à 16,
**caractérisé en ce qu'**il est prévu au moins un élément de sécurité (73), en particulier une vis de sécurité, déplaçable depuis l'extérieur en traversant la paroi latérale de la broche (11) pour pénétrer dans son perçage longitudinal (35), avec lequel l'organe de serrage (63) peut être fixé dans sa position de départ par rapport à la broche (11) avant actionnement du dispositif de réglage (67).
